# EUROPÄISCHE PATENTSCHRIFT

(11) **EP 2 293 713 B1**
(45) Veröffentlichungstag und Bekanntmachung des Hinweises auf die Patenterteilung: **29.08.2012**
(21) Anmeldenummer: 09772371.2
(22) Anmeldetag: 26.06.2009
(51) Int. Cl.: A61B 3/16, A61F 2/16, A61B 5/00, A61F 2/14

(54) **KAPSELSPANNRING MIT SPULE FÜR DIE INDUKTIVE KOPPLUNG AN EIN EXTERNES ELEKTROMAGNETISCHES FELD**
CAPSULE CLAMP RING WITH COIL FOR INDUCTIVE COUPLING TO AN EXTERNAL ELECTROMAGNETIC FIELD
BAGUE DE SERRAGE CAPSULAIRE AVEC BOBINE POUR L ACCOUPLEMENT INDUCTIF À UN CHAMP ÉLECTROMAGNÉTIQUE EXTÉRIEUR

(30) Priorität: 03.07.2008 DE 102008040142
(43) Veröffentlichungstag der Anmeldung: 16.03.2011
(73) Patentinhaber: Robert Bosch GmbH, 70442 Stuttgart (DE)
(72) Erfinder: SCHOLTEN, Dick, 70188 Stuttgart (DE)
(86) Internationale Anmeldenummer: PCT/EP2009/058011
(87) Internationale Veröffentlichungsnummer: WO 2010/000671

(56) Entgegenhaltungen:
- WO-A-01/21063
- WO-A2-2005/117748
- DE-C1- 10 215 729

## Beschreibung

### Stand der Technik

Die Erfindung betrifft einen geschlossenen Kapselspannring.

Kapselspannringe dienen zur Stabilisierung des Kapselsacks im Auge und werden als Implantat in den Kapselsack eingesetzt, beispielsweise nach Entfemen der natürlichen Linse eines Auges zur Stützung des Kapselgewebes. Nach Entfernen der natürliche Linse, beispielsweise aufgrund einer starken Trübung, ist es erforderlich, dass der eröffnete Kapselsack im Wesentlichen in seiner ursprünglichen Form verbleibt und dadurch die Implantation einer künstlichen intraokularen Linse erleichtert werden kann.

Bedingt durch einen erhöhten mittleren Augeninnendruck, beispielsweise bei der Augenerkrankung Glaukom, sterben die Nervenfasem der Retina ab. Vielfach kann der Augeninnendruck mit medikamentösen Augentropfen auf einen akzeptablen Wert reduziert werden. Für eine optimale Langzeittherapie muss jedoch die Dosierung der medikamentösen Augentropfen an den mittleren Augeninnendruck angepasst werden. Der Augeninnendruck kann zwar relativ genau von einem Augenarzt gemessen werden, allerdings ist eine sporadische Messung in vielen Fällen unzureichend, da der Augeninnendruck unter anderem von den Faktoren Körperlage, Tageszeit und/oder Stress abhängig ist.

Aus der DE 197 28 069 C1 ist eine Integration eines Drucksensors zur Messung des Augeninnendrucks in einer Kunstlinse beschrieben. Die beschriebene Vorrichtung weist eine Fernmesseinrichtung auf, welche einen Drucksensor, einen die Sensorsignale zwischenspeichernden Datalogger, einen die Sensorsignale in drahtlos übertragbare Informationen wandelnde Einrichtung und eine Sendeeinrichtung enthält Ferner ist eine außerhalb des Auges angeordnete Empfangseinrichtung beschrieben, welche die aus dem Datalogger zeitlich begrenzt abgefragten Informationen von der Sendeeinrichtung empfängt und an eine Auswerteeinrichtung angeschlossen ist, in welcher die empfangenen Informationen in Daten des Augeninnendrucks für eine Aufzeichnung gewandelt werden.

Diese Konstruktion sowie andere aus dem Stand der Technik bekannte Konstruktionen erlauben nur eine sehr eingeschränkte Auswahl an möglichen Kunstlinsen, in die ein derartiger Drucksensor zur Messung des Augeninnendrucks integriert werden kann.

Aus der WO 2005/11748 A2 ist ein Kapseläquatorring bekannt, welcher nach dem Entfernen einer natürlichen Linse in den Kapselsack eines Auges implantierbar ist und im implantierten Zustand mit seinem Außenumfang an der Innenseite des Kapselsacks im Wesentlichen an dessen Äquator anliegt und den Kapselsack radial stabilisiert.

### Offenbarung der Erfindung

Anspruch 1 offenbart den Gegenstand der Erfindung.

Der erfindungsgemäße geschlossene Kapselspannring weist demgegenüber den Vorteil auf, dass er ohne Beschränkung auf eine bestimmte Kunstlinse oder eine bestimmte Kunstlinsenform eingesetzt werden kann.

Erfindungsgemäß wird dies erreicht durch einen geschlossenen Kapselspannring, mit wenigstens eine Windung aufweisende Spule, wobei die Windung in dem Kapselspannring angeordnet ist

Erfindungsgemäß ist damit ein geschlossener Kapselspannring gegeben, welcher beispielsweise nach einer Katarakt-Operation benötigt wird. Mittels einer minimal invasiven Implantation kann mit dem erfindungsgemäßen geschlossenen Kapselspannring der Kapselsack im Auge in Form gehalten werden. Zusätzlich zu dem Kapselspannring kann eine beliebige Kunstlinse in das Auge eingesetzt werden.

Weiterhin ist es gemäß einer bevorzugten Weiterbildung der Erfindung vorgesehen, dass der geschlossene Kapselspannring aus wenigstens einem flexiblen Segment und wenigstens einem steifen Segment ausgebildet ist, wobei je ein flexibles Segment und je ein steifes Segment abwechselnd angeordnet sind.

Erfindungsgemäß ist vorgesehen, dass der Kapselspannring eine Einrichtung zur Messung und/oder zur Steuerung einer physikalischen Größe aufweist, wobei die Spule eine Verbindung mit der Einheit aufweist. Gemäß einer weiteren bevorzugten Weiterbildung der Erfindung ist vorgesehen, dass die Einrichtung als Druckmesseinheit zur Messung des intraokularen Drucks ausgeführt ist Als Druckmesseinheit eignen sich derartige Sensoren, mit denen der Augeninnendruck erfasst werden kann, sowie andere aus dem Stand der Technik bekannte Sensoren für den Einsatz in Blutgefäßen und als Herzkatheder, welche vorzugsweise in Oberflächenmikromechanik ausgebildet sind. Dadurch, dass ein geschlossener Kapselspannring vorgesehen ist, wird es ermöglicht, eine Spule mit ein oder mehreren Windungen zu integrieren, welche in Verbindung mit der Druckmesseinheit stehen. Dadurch wird es ermöglicht, den intraokularen Druck zu messen, ohne auf eine bestimmte Kunstlinsenform beschränkt zu sein. Weiterhin kann vorgesehen sein, andere physikalische Größen wie Temperatur oder chemische Substanzen mittels der Einheit zu messen. Ebenso kann durch die Einheit ein Medikament dosiert werden.

Eine bevorzugte Weiterbildung der Erfindung liegt ferner darin, dass das flexible Segment faltbar und/oder knickbar ausgeführt ist. Dadurch wird es ermöglicht, dass der Kapselspannring zusammenfaltbar ist Ganz besonders ist es gemäß einer bevorzugten Weiterbildung der Erfindung vorgesehen, dass der Kapselspannring entlang einer Falzlinie zusammenfaltbar ist. Weiterhin ist es bevorzugt, dass der Kapselspannring vorzugsweise entlang der Äquatorlinie zusammenfaltbar ist und somit im zusammengefalteten Zustand eine halbkreisartige Form aufweisen kann. In dieser zusammengefalteten Form kann der Kapselspannring besonders einfach mittels einer minimalen-invasiven Implantation in das Auge implantiert werden. Mit anderen Worten kann der Kapselspannring mittels einer derartigen minimal-invasiven Implantation durch eine Öffnung in das Auge "eingedreht" werden.

Weiterhin ist es bevorzugt, dass die Spule zur Energieversorgung und/oder zur Datenübertragung der Einheit und/oder zur Datenübertragung der von der Einheit gemessenen physikalischen Größe vorgesehen ist. Vorzugsweise erfolgen die Energieversorgung und/oder die Datenübertragung induktiv.

Grundsätzlich kann die Einheit an einer beliebigen Position auf/in dem Kapselspannring angeordnet sein. Gemäß einer bevorzugten Weiterbildung der Erfindung ist jedoch vorgesehen, dass die Einheit auf/in dem flexiblen Segment angeordnet ist. Beispielsweise kann die Einheit durch das Aufbringen von Silikon verkapselt werden.

Gemäß einer bevorzugten Weiterbildung der Erfindung ist vorgesehen, dass zwei flexible Segmente und zwei steife Segmente vorgesehen sind. Weiterhin ist bevorzugt, dass die Länge des steifen Segmentes größer ist als die Länge des flexiblen Segmentes. Vorzugsweise ist die Länge des flexiblen Segmentes derart bemessen, dass dieses das Zusammenfalten des Kapselspannrings erlaubt, während die Länge des steifen Segmentes den größten Teil des Umfangs des Kapselspannrings ausmacht. Gemäß der vorliegenden Weiterbildung der Erfindung, also bei zwei steifen Segmenten, beträgt die Länge des steifen Segmentes vorzugsweise im Wesentlichen die Hälfe des Umfangs des Kapselspannrings. Dadurch kann eine hohe Stabilität des Kapselspannrings erreicht werden.

Weiterhin ist es gemäß einer bevorzugten Weiterbildung der Erfindung vorgesehen, dass drei steife Segmente vorgesehen sind, wobei die Länge des ersten steifen Segmentes größer ist als die Summe der Längen des zweiten steifen Segmentes und des dritten steifen Segmentes. Ganz besonders bevorzugt entspricht die Länge des ersten steifen Segmentes im Wesentlichen dem halben Umfang des Kapselspannringes, und die Länge des zweiten steifen Segmentes und des dritten steifen Segmentes entspricht im Wesentlichen je einem Viertel des Umfangs des Kapselspannrings.

Wie zuvor gesagt worden ist, kann die Einheit an einer beliebigen Position auf/in dem Kapselspannring angeordnet sein. Gemäß einer bevorzugten Weiterbildung der Erfindung ist jedoch vorgesehen, dass drei flexible Segmente vorgesehen sind, wobei die Einheit auf/in dem flexiblen Segment angeordnet ist, welche zwischen dem zweiten steifen Segment und dem dritten steifen Segment angeordnet ist. Somit kann der Kapselspannring entlang der Falzlinie, welche vorzugsweise im Wesentlichen mit dem Äquator des Kapselspannrings übereinstimmt, zusammengefaltet werden, ohne dass die Faltung des Kapselspannrings unter Einbeziehung des flexiblen Segmentes erfolgt, in dem die Druckmesseinheit angeordnet ist. Ein derartiger zusammengefalteter Kapselspannring kann in das Auge minimal-invasiv implantiert werden, wobei sich vorzugsweise der Kapselspannring nach der Implantation im Auge entfaltet.

Eine bevorzugte Weiterbildung liegt ferner darin, dass das steife Segment aus Polymethylmethacrylat (PMMA) ausgeführt ist. Vorzugsweise ist das flexible Segment aus Hydroxyethylmethacrylat-co-Methylmethacrylat (HEMA/MMA) oder Silikon ausgeführt. Ferner kann das flexible Segment auch hydrophile Eigenschaften aufweisen. Dadurch wird die Falt- und/oder Knickbarkeit des Kapselspannrings verbessert, so dass das Implantieren des Kapselspannrings in den eröffneten Kapselsack erleichtert ist

Die Erfindung betrifft ferner eine Vorrichtung zur Messung des intraokularen Drucks mit einem erfindungsgemäßen geschlossenen Kapselspannrings und einer außerhalb des Auges vorgesehenen externen Einrichtung zur Energieversorgung der Spule und zur Datenübertragung mit der Spule. Mit anderen Worten kann die externe Einrichtung die Spule der des geschlossenen Kapselspannrings mit induktiv übertragener Energie versorgen, so dass beispielsweise die in Verbindung mit der Spule stehende Druckmesseinheit den Augeninnendruck messen kann. Der von der Druckmesseinheit gemessene Augeninnendruck kann dann über die Spule induktiv an die externe Einrichtung übertragen werden. Die induktiv übertragenen Daten der Druckmesseinheit können auf einer Datenauswerteeinrichtung gespeichert, analysiert und/oder visualisiert werden.

Der zuvor beschriebene geschlossenen Kapselspannring und/oder die Vorrichtung zur Messung des intraokularen Drucks finden vorzugsweise Anwendung in der Medizintechnik, z. B. bei der schon angesprochenen Augenerkrankung Glaukom. Weitere Anwendungsgebiete liegen in der intraokularen Druckmessung für andere medizinische Einsatzbereiche.

Nachfolgend wird die Erfindung anhand bevorzugter Ausführungsbeispiele unter Bezugnahme auf die Zeichnung im Detail beschrieben. In der Zeichnung zeichnen:
- Fig. 1: eine schematische Querschnittsdarstellung eines geschlossenen Kapselspannrings gemäß einem bevorzugten Ausführungsbeispiel der Erfindung, und
- Fig. 2: eine schematische Querschnittsdarstellung des geschlossenen Kapselspannrings gemäß einem weiteren bevorzugten Ausführungsbeispiel der Erfindung.

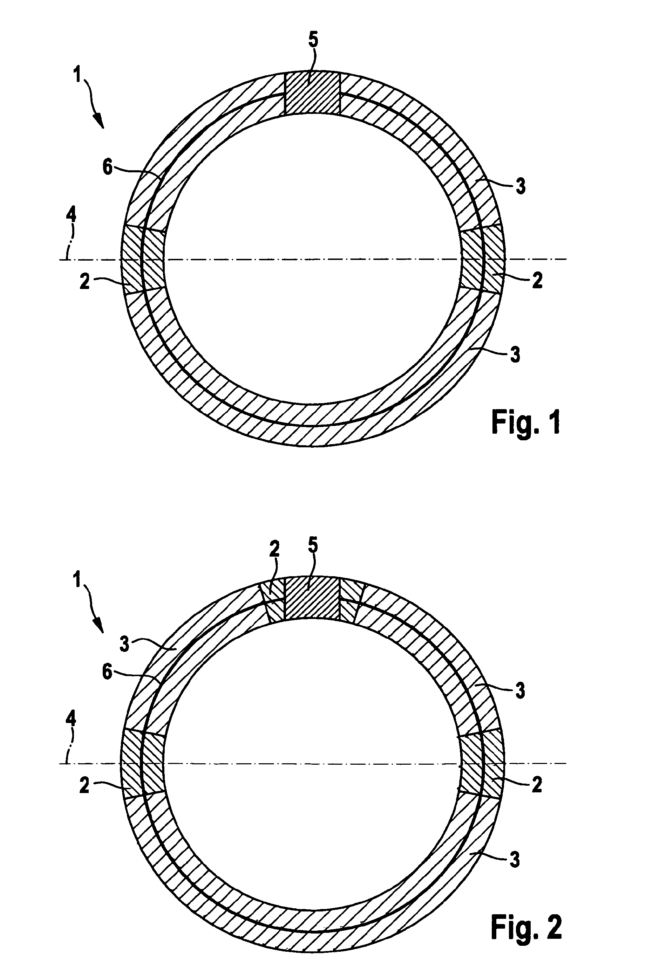

Aus den Figuren 1 und 2 ist jeweils eine schematische Querschnittsdarstellung eines geschlossenen Kapselspannrings 1 gemäß einem bevorzugten Ausführungsbeispiel der Erfindung ersichtlich. Der in Fig. 1 ersichtliche Kapselspannring 1 weist zwei flexible Segmente 2 und zwei steife Segmente 3 auf, wobei je ein flexibles Segment 2 und je ein steifes Segment 3 abwechselnd angeordnet sind.

Gemäß dem bevorzugten Ausführungsbeispiel der Erfindung sind die flexiblen Segmente 2 falt- und/oder knickbar ausgeführt, so dass der Kapselspannring 1 entlang einer Falzlinie 4 zusammenfaltbar ist Ein derartig zusammengefalteter Kapselspannring 1 kann durch eine Öffnung in ein Auge mittels einer minimal-invasiven Implantation eingebracht werden. Vorzugsweise entfaltet sich der Kapselspannring 1 nach der Implantation im Auge.

Grundsätzlich können die flexiblen Segmente 2 und die steifen Segmente 3 aus beliebigen Materialien ausgeführt sein. Gemäß dem bevorzugten Ausführungsbeispiel der Erfindung ist jedoch bevorzugt, dass das flexible Segment 2 aus Hydroxyethylmethacrylat -Co-Methylmethacrylat (HEMA/MMA) oder Silikon ausgeführt ist. Das steife Segment 3 ist vorzugsweise aus Polymethylmethacrylat (PMMA) ausgeführt.

Wie aus Fig. 1 ersichtlich, stimmt die Länge des steifen Segmentes 3 im Wesentlichen mit dem halben Umfang des Kapselspannrings 1 überein. Dadurch wird eine hohe Stabilität des Kapselspannrings 1 erreicht

Der geschlossene Kapselspannring weist ferner eine Einheit 5 auf, welche, wie aus Fig. 1 ersichtlich, in dem steifen Segment 3 des Kapselspannrings 1 angeordnet ist und gemäß dem vorliegenden Ausführungsbeispiel der Erfindung als Druckmesseinheit ausgeführt ist. Mittels der Druckmesseinheit ist der intraokulare Druck messbar. Gemäß dem vorliegenden Ausführungsbeispiel der Erfindung ist eine in Verbindung mit der Druckmesseinheit stehenden und wenigstens eine Windung 6 aufweisende Spule, nicht gezeigt, vorgesehen. Die Spule dient zur Energieversorgung der Druckmesseinheit und zur Datenübertragung des von der Druckmesseinheit gemessenen intraokularen Drucks. Die Energieversorgung erfolgt vorzugsweise induktiv durch eine externe Einrichtung, wobei die externe Einrichtung auch zur induktiven Datenübertragung mit der Spule vorgesehen ist.

Gemäß einem weiteren bevorzugten Ausführungsbeispiel der Erfindung weist der geschlossene Kapselspannring drei flexible Segmente 2 und drei steife Segmente 3 auf, wie aus Fig. 2 ersichtlich. Gemäß diesem bevorzugten Ausführungsbeispiel der Erfindung ist die Druckmesseinheit in dem flexiblen Segment 2 vorgesehen. Vorzugsweise stimmt die Länge des ersten steifen Segmentes 3 im Wesentlichen mit dem halben Umfang des Kapselspannrings 1 überein, während die Länge des zweiten steifen Segmentes 3 und des dritten steifen Segmentes 3 im Wesentlichen mit der Länge eines Viertels des Umfangs des Kapselspannrings 1 übereinstimmt. Somit stimmt auch die Falzlinie 4 im Wesentlichen mit dem Äquator des Kapselspannrings 1 überein.

Wie weiterhin aus Fig. 2 ersichtlich, ist die Druckmesseinheit in dem flexiblen Segment 2 vorgesehen, welches zwischen den beiden steifen Segmenten 3 angeordnet ist, die im Wesentlichen in ihrer Länge mit einem Viertel des Kapselspannrings 1 übereinstimmen.

## Patentansprüche

1. Geschlossener Kapselspannring (1), mit wenigstens eine Windung (6) aufweisende Spule, wobei die Windung (6) in dem Kapselspannring (1) angeordnet ist, **dadurch gekennzeichnet, dass** der Kapselspannring (1) eine Einrichtung (5) zur Messung und/oder zur Steuerung einer physikalischen Größe aufweist, wobei die Spule eine Verbindung mit der Einrichtung (5) aufweist.

2. Geschlossener Kapselspannring nach Anspruch 1, wobei wenigstens ein flexibles Segment (2) und wenigstens ein steifes Segment (3) vorgesehen sind, wobei je ein flexibles Segment (2) und je ein steifes Segment (3) abwechselnd angeordnet sind.

3. Geschlossener Kapselspannring nach Anspruch 1 oder 2, wobei die Einrichtung (5) als Druckmesseinheit zur Messung des intraokularen Drucks ausgeführt ist

4. Geschlossener Kapselspannring nach einem der Ansprüche 1 bis 3, wobei das flexible Segment (2) faltbar und/oder knickbar ausgeführt ist

5. Geschlossener Kapselspannring nach einem der Ansprüche 1 bis 4, wobei der Kapselspannring (1) entlang einer Falzlinie (4) zusammenfaltbar ist.

6. Geschlossener Kapselspannring nach einem der Ansprüche 1 bis 5, wobei die Spule zur Energieversorgung und/oder Datenübertragung der Einrichtung (5) und/oder zur Datenübertragung der von der Einrichtung (5) gemessenen physikalischen Größe vorgesehen ist.

7. Geschlossener Kapselspannring nach einem der Ansprüche 1 bis 6, wobei die Einrichtung (5) auf/in dem flexiblen Segment (2) angeordnet ist.

8. Geschlossener Kapselspannring nach einem der Ansprüche 1 bis 7, wobei zwei flexible Segmente (2) und zwei steife Segmente (3) vorgesehen sind.

9. Geschlossener Kapselspannring nach Anspruch 8, wobei die Länge des steifen Segmentes (3) größer ist als die Länge des flexiblen Segmentes (2).

10. Geschlossener Kapselspannring nach einem der Ansprüche 1 bis 9, wobei drei steife Segmente (3) vorgesehen sind, wobei die Länge des ersten steifen Segmentes (3) größer ist als die Summe der Längen des zweiten steifen Segmentes (3) und des dritten steifen Segmentes (3).

11. Geschlossener Kapselspannring nach Anspruch 10, wobei drei flexible Segmente (2) vorgesehen sind, wobei die Einrichtung (5) auf dem flexiblen Segment (2) angeordnet ist, welche zwischen dem zweiten steifen Segment (3) und dem dritten steifen Segment (3) angeordnet ist

12. Geschlossener Kapselspannring nach einem der Ansprüche 1 bis 11, wobei das steife Segment (3) aus Polymethylmethacrylat ausgeführt ist

13. Vorrichtung zur Messung des intraokularen Drucks mit einem geschlossenem Kapselspannring nach einem der Ansprüche 1 bis 12, und einer außerhalb des Auges vorgesehenen externen Einrichtung zur Energieversorgung der Spule und zur Datenübertragung mit der Spule.

## Claims

1. Closed capsular tension ring (1), comprising a coil having at least one turn (6), the turn (6) being arranged in the capsular tension ring (1), **characterized in that** the capsular tension ring (1) has a device (5) for measuring and/or controlling a physical variable, the coil having a connection to the device (5).

2. Closed capsular tension ring according to Claim 1, at least one flexible segment (2) and at least one rigid segment (3) being provided, a flexible segment (2) and a rigid segment (3) being respectively arranged in an alternating sequence.

3. Closed capsular tension ring according to Claim 1 or 2, the device (5) being designed as a pressure measuring unit for measuring intraocular pressure.

4. Closed capsular tension ring according to one of Claims 1 to 3, the flexible segment (2) being designed such that it can be folded and/or bent.

5. Closed capsular tension ring according to one of Claims 1 to 4, the capsular tension ring (1) being able to be folded together along a folding line (4).

6. Closed capsular tension ring according to one of Claims 1 to 5, the coil being intended for the power supply to and/or data transmission to or from the device (5) and/or for the data transmission of the physical variable measured by the device (5).

7. Closed capsular tension ring according to one of Claims 1 to 6, the device (5) being arranged on/in the flexible segment (2).

8. Closed capsular tension ring according to one of Claims 1 to 7, two flexible segments (2) and two rigid segments (3) being provided.

9. Closed capsular tension ring according to Claim 8, the length of the rigid segment (3) being greater than the length of the flexible segment (2).

10. Closed capsular tension ring according to one of Claims 1 to 9, three rigid segments (3) being provided, the length of the first rigid segment (3) being greater than the sum of the lengths of the second rigid segment (3) and the third rigid segment (3).

11. Closed capsular tension ring according to Claim 10, three flexible segments (2) being provided, the device (5) being arranged on the flexible segment (2) that is arranged between the second rigid segment (3) and the third rigid segment (3).

12. Closed capsular tension ring according to one of Claims 1 to 11, the rigid segment (3) being made of polymethylmethacrylate.

13. Device for measuring intraocular pressure with a closed capsular tension ring according to one of Claims 1 to 12, and an external device, provided outside the eye, for the power supply to the coil and for the data transmission with the coil.

## Revendications

1. Anneau fermé (1) de serrage de capsule, présentant une bobine dotée d'au moins un enroulement (6), l'enroulement (6) étant disposé dans l'anneau (1) de serrage de capsule,
**caractérisé en ce que**
l'anneau (1) de serrage de capsule présente un dispositif (5) de mesure et/ou de commande d'une grandeur physique, la bobine présentant un raccordement avec le dispositif (5).

2. Anneau fermé de serrage de capsule selon la revendication 1, dans lequel au moins un segment flexible (2) et au moins un segment rigide (3) sont prévus, un segment flexible (2) et un segment rigide (3) étant disposés chaque fois en alternance.

3. Anneau fermé de serrage de capsule selon les revendications 1 ou 2, dans lequel le dispositif (5) est configuré comme unité de mesure de pression destinée à mesurer la pression intraoculaire.

4. Anneau fermé de serrage de capsule selon l'une des revendications 1 à 3, dans lequel le segment flexible (2) peut être replié et/ou coudé.

5. Anneau fermé de serrage de capsule selon l'une des revendications 1 à 4, dans lequel l'anneau (1) de serrage de capsule peut être replié le long d'une ligne de pliage (4).

6. Anneau fermé de serrage de capsule selon l'une des revendications 1 à 5, dans lequel la bobine est prévue pour fournir de l'énergie et/ou transmettre des données du dispositif (5) et/ou transmettre des données de la grandeur physique mesurée par le dispositif (5).

7. Anneau fermé de serrage de capsule selon l'une des revendications 1 à 6, dans lequel le dispositif (5) est disposé sur ou dans le segment flexible (2).

8. Anneau fermé de serrage de capsule selon l'une des revendications 1 à 7, dans lequel deux segments flexibles (2) et deux segments rigides (3) sont prévus.

9. Anneau fermé de serrage de capsule selon la revendication 8, dans lequel la longueur du segment rigide (3) est supérieure à la longueur du segment flexible (2).

10. Anneau fermé de serrage de capsule selon l'une des revendications 1 à 9, dans lequel trois segments rigides (3) sont prévus, la longueur du premier segment rigide (3) étant supérieure à la somme des longueurs du deuxième segment rigide (3) et du troisième segment rigide (3).

11. Anneau fermé de serrage de capsule selon la revendication 10, dans lequel trois segments flexibles (2) sont prévus, le dispositif (5) étant disposé sur le segment flexible (2) disposé entre le deuxième segment rigide (3) et le troisième segment rigide (3).

12. Anneau fermé de serrage de capsule selon l'une des revendications 1 à 11, dans lequel le segment rigide (3) est réalisé en poly(méthacrylate de méthyle).

13. Dispositif de mesure de la pression intraoculaire à l'aide d'un anneau fermé de serrage de capsule selon l'une des revendications 1 à 12 et d'un dispositif externe prévu à l'extérieur de l'oeil et destiné à alimenter la bobine en énergie et à échanger des données avec la bobine.
